# EUROPEAN PATENT SPECIFICATION

(11) **EP 3 103 468 B1**
(45) Date of publication and mention of the grant of the patent: **15.07.2020**
(21) Application number: 16164865.4
(22) Date of filing: 12.10.2006
(51) Int. Cl.: A61K 38/13, A61K 45/06, A61P 27/02

(54) **PREVENTION OF DRUG-ASSOCIATED KERATOCONJUNCTIVITIS WITH A CYCLOSPORIN**
PRÄVENTION VON MIT ARZNEIMITTELN IN ZUSAMMENHANG STEHENDEN KERATOKONJUNCTIVITIS MIT CYCLOSPORIN
PRÉVENTION DE LA KÉRATOCONJONCTIVITE ASSOCIÉE À UN MÉDICAMENT GRÂCE À UNE CYCLOSPORINE

(30) Priority: 14.10.2005 US 596709; 30.11.2005 US 597431 P; 22.06.2006 US 805509 P; 11.10.2006 US 548631
(43) Date of publication of application: 14.12.2016
(62) Divisional of application: 09170213.4
(73) Proprietor: ALLERGAN, INC., Irvine, CA 92612 (US)
(72) Inventor: Schiffman, Rhett M., Laguna Beach, CA California 92651 (US); Barnett, Pamela S., Aliso Viejo, CA California 92656 (US); Feinermann, Gregg, Irvine, CA California 92604 (US); Barth, Neil, Newport Beach, CA California 92660 (US)
(74) Representative: Hoffmann Eitle

(56) References cited:
- US-A- 4 839 342
- US-A- 5 411 952
- CALONGE MARGARITA: "The Treatment of Dry Eye", SURVEY OF OPHTHALMOLOGY, SURVEY OF OPHTHALMOLOGY INC, XX, vol. 45, no. Suppl.2, March 2001 (2001-03) , pages 227-239, XP002444863, ISSN: 0039-6257, DOI: 10.1016/S0039-6257(00)00205-8
- FRAUNFELDER FREDERICK T. ET AL: "The Role of Medications in Causing Dry Eye", JOURNAL OF OPHTHALMOLOGY, vol. 99, no. 2, January 2012 (2012-01), pages 197-8, XP055300785, US ISSN: 2090-004X, DOI: 10.1016/j.ophtha.2003.03.001

## Description

### CROSS-REFERENCE TO RELATED APPLICATIONS

This application claims priority to: United States Provisional Patent Application No. 60/596,709, filed on October 14, 2005; United States Provisional

Patent Application No. 60/597,431, filed on November 30, 2005; and United States Provisional Patent Application No. 60/805,509, filed on June 22, 2006.

### DESCRIPTION OF THE INVENTION

Patients undergoing treatment with certain therapeutically active agents can have certain ocular conditions as a result of that treatment. In particular, patients undergoing chemotherapy with a therapeutically active agent effective for treatment of a cancer often have ocular conditions as a result of that treatment.

The invention id directed to a cyclosporin or derivative thereof, or a combination thereof for use in advance of treatment with a therapeutically active agent, said therapeutically active agent being an chemotherapy agent or an antiviral agent or an immunomodulator, in a method of prophylactically treating keratoconjunctivitis associated with the use of said therapeutically active agent.

"Administration of a therapeutically active agent to said mammal" means administration of the therapeutically active agent to the mammal in any way that a therapeutically active agent may be administered. Thus, administration of the therapeutically active agent is not limited to the eye, but may include systemic administration via oral, intravenous, rectal, or other means; or administration locally to any part of the body by injection, implantation, topical administration, or other means.

Administration of the therapeutically active agent need not exactly overlap in time with the administration of the cyclosporin or derivative thereof, or a combination thereof. In the invention, the cyclosporin or derivative thereof, or a combination thereof is administered to a mammal before the mammal receives any of the therapeutically active agent to avoid the onset of the ocular condition. The cyclosporin or derivative thereof, or a combination thereof, might continue to be administered after the mammal has begun to receive the therapeutically active agent. The cyclosporin or derivative thereof, or a combination thereof, might also continue to be administered after the mammal has ceased receiving the therapeutically active agent. Administration of the cyclosporin or derivative thereof, or a combination thereof might also be continued such that it is simultaneous with the administration of the therapeutically active agent. Any time relationship may exist between the mammal receiving the therapeutically active agent and the cyclosporin or derivative thereof, or a combination thereof, provided that the latter is used in advance of the former, and that the use of the latter is reasonably related to prophylactically treating keratoconjunctivitis associated with the former.

It may be convenient to provide a single pharmaceutical composition which comprises both (i) the cyclosporin or derivative thereof, or a combination thereof and (ii) the therapeutically active agent during any time period that the agents are to be administered simultaneously.

It may be convenient to provide (i) the cyclosporin or derivative thereof, or a combination thereof and (ii) the therapeutically active agent in form of a kit. For example, the agents may be packaged together. For example, (i) the cyclosporin or derivative thereof, or a combination thereof and (ii) the therapeutically active agent may each be packaged in conventional pharmaceutical packaging such as boxes, jars, blister packs, vials, bottles, syringes etc., and the individually packaged components may then be combined to form a kit e.g. by the use of further packaging such as a box, or by joining up the individual packages. When in kit form, the agents can be taken independently of one another, thus allowing the user freedom to decide the temporal relationship between his use of each of the agents.

A cyclosporin or derivative thereof, including cyclosporin A, for use in the prophylactic treatment of ocular conditions occurring in a person who will undergo treatment with a therapeutically active agent for the treatment of cancer as defined in claim 1 is contemplated. Accordingly, a particular patient group which may benefit from the present invention is that of persons who may suffer from keratoconjunctivitis resulting from the use of a chemotherapy agent.

Also contemplated is a cyclosporin or derivative thereof, including cyclosporin A, for use in the prophylactic treatment of ocular conditions occurring in a person who will undergo treatment with an antiviral agent. Accordingly, a particular patient group which may benefit from the present invention is that of persons who may suffer from keratoconjunctivitis resulting from the use of an antiviral agent.

Also contemplated is a cyclosporin or derivative thereof, including cyclosporin A, for use in the prophylactic treatment of ocular conditions occurring in a person who will undergo treatment with an immunomodulator. Accordingly, a particular patient group which may benefit from the present invention is that of persons who may suffer from keratoconjunctivitis resulting from the use of an immunomodulator.

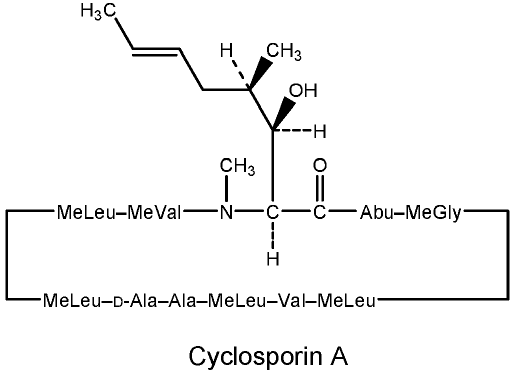

Cyclosporin A is a cyclic peptide with immunosuppressive properties having the structure shown above. It is also known by other names including cyclosporine, cyclosporine A, ciclosporin, and ciclosporin A.

Other cyclosporins include cyclosporine b, cyclosporine D, cyclosporine G, which are well known in the art. Cyclosporin derivatives are also known in the art. For example, United States Patent Nos. 6,254,860 and 6,350,442, illustrate several examples.

The ocular condition to be prevented is keratoconjunctivitis.

Also contemplated is a cyclosporin A for use in a method comprising administering topically to the eye of a person in advance of treatment with docetaxel, wherein said method is effective in prophylactically treating an keratoconjunctivitis associated with the administration of docetaxel.

Although the ocular condition may be associated with any antiviral agent, the following antiviral agents are contemplated in particular:
Zalcitabine, and
Rimantadine Hydrochloride.

Although the ocular condition may be associated with any chemotherapy agent, the following
chemotherapy agents are contemplated in particular:
Paclitaxel and derivatives thereof, such as Docetaxel
Doxorubicin Hydrochloride,
Irinotecan Hydrochloride,
Fluorouracil,
Imatinib Mesylate, and
Rituximab.

Derivatives of paclitaxel generally include the macrocycle shown below, where derivatives are formed at a hydroxyl moiety.

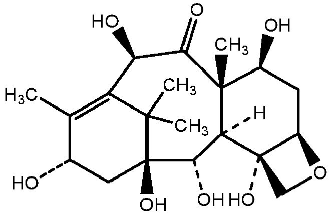

Chemotherapeutic compounds incorporating this structure are thus contemplated. For example, the structures of paclitaxel and docetaxel are shown below.

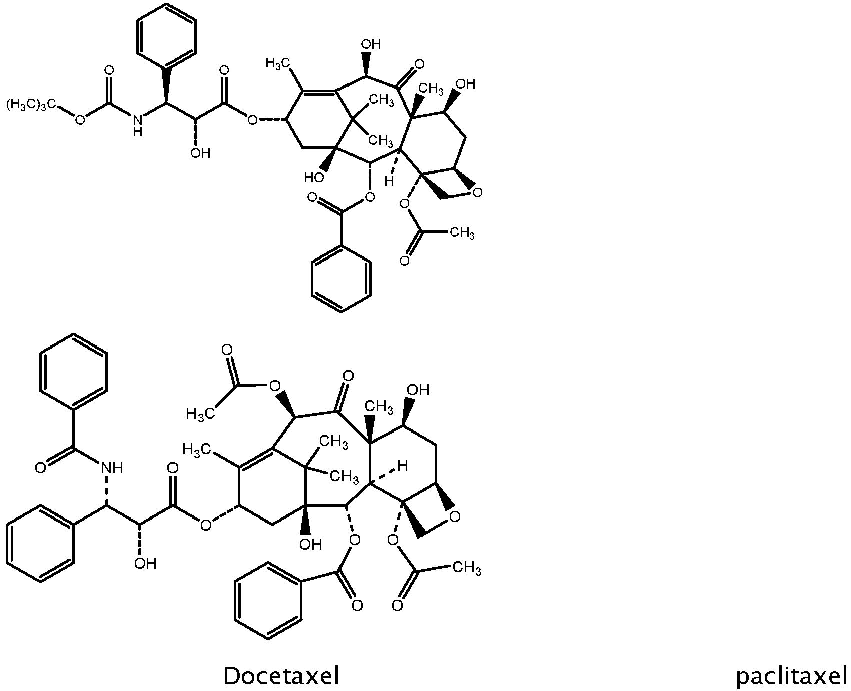

In one embodiment, the chemotherapy agent is docetaxel.

Although the ocular condition may be associated with any immunomodulator, the following
immunomodulators are contemplated in particular:
Interferon alfa-2b, Recombinant
Mycophenolate Mofetil, and
Mycophenolate Mofetil Hydrochloride.

The following therapeutically active agents may cause lacrimal duct stenosis:
docetaxel.

The following therapeutically active agents may cause lacrimation:
interferon alfa-2b, recombinant,
doxorubicin hydrochloride,
irinotecan hydrochloride,
fluorouracil,
docetaxel, and
zalcitabine.

The following therapeutically active agents may cause abnormal lacrimation:
mycophenolate motefil,
mycophenolate motefil hydrochloride,
imatinib mesylate,
ritumixab, and
rimantadine hydrochloride.

The following therapeutically active agents may cause keratitis:
Amantadine Hydrochloride,
Erlotinib,
Bexarotene, and
Voriconazole.

The following therapeutically active agents may cause keratoconjunctivitis:
Capecitabine.

The following therapeutically active agents may cause conjunctivitis:
Risedronate Sodium,
Leflunomide,
Mycophenolate Mofetil,
Oxaliplatin,
Cetuximab,
Ribavirin,
Rituximab,
Basiliximab,
Erlotinib,
Capecitabine,
Doxorubicin Hydrochloride,
Imiquimod,
Amphotericin B, liposomal,
Zolpidem Tartrate,
Glatiramer Acetate,
Epirubicin Hydrochloride,
Saquinavir,
Enfuvirtide,
Imatinib Mesylate,
Gefitinib,
Lamotrigine,
Delavirdine Mesylate,
Rituximab,
Ivermectin,
Palivizumab,
Oseltamivir Phosphate,
Bexarotene,
Docetaxel,
Abacavir Sulfate,
Lamivudine,
Zidovudine,
Voriconazole,
Nevirapine,
Ribavirin, and
Abacavir Sulfate.

Additionally, one or more of the ocular conditions disclosed herein may be associated with the following therapeutically active agents: abacavir sulfate, amantadine hydrochloride, amphotericin B, basiliximab, bexarotene, capecitabine, cetuximab, delavirdine mesylate, docetaxel, doxorubicin hydrochloride, enfuvirtide, epirubicin hydrochloride, erlotinib, fluorouracil, gefitinib, glatiramer acetate, imatinib mesylate, imiquimod, interferon alfa-2b, irinotecan hydrochloride, ivermectin, lamivudine, lamotrigine, leflunomide, mycophenolate mofetil, mycophenolate mofetil hydrochloride, nevirapine, oseltamivir phosphate, oxaliplatin, palivizumab, ribavirin, rimantadine hydrochloride, risedronate sodium, rituximab, saquinavir, voriconazole, zalcitabine, zidovudine, and zolpidem tartrate.

The therapeutically active agent is administered in the usual manner known in the art for the condition being treated.

A therapeutically active agent and cyclosporin A may be administered in a single composition after the prophylactic treatment with cyclosporin in advance of treatment with the therapeutically active agent has taken place.

Useful compositions are disclosed in the following patent applications: United States Patent Application Serial No. 11/181, 409, filed on July 13, 2005; United States Patent Application Serial No. 11/181, 509, filed on July 13, 2005; United States Patent Application Serial No. 11/181, 187, filed on July 13, 2005; United States Patent Application Serial No. 11/181, 178, filed on July 13, 2005; United States Patent Application Serial No. 11/181, 428, filed on July 13, 2005; United States Patent Application Serial No. 11/255,821, filed on October 19, 2005; United States Patent Application Serial No. 11/161,218, filed on July 27, 2005; and United States Provisional Patent Application Serial Number 60/727,684, filed on October 17, 2005.

In one embodiment, cyclosporin A is administered in the form of Restasis®, available from Allergan, Inc. The cyclosporin A is administered twice a day as indicated on the package insert.

## Claims

1. A cyclosporin or derivative thereof, or a combination thereof, for use in advance of treatment with a therapeutically active agent, said therapeutically active agent being a chemotherapy agent, an antiviral agent or an immunomodulator, in a method of prophylactically treating keratoconjunctivitis associated with the use of said therapeutically active agent.

2. The cyclosporin for use according to claim 1, wherein said cyclosporin is cyclosporin A.

## Patentansprüche

1. Cyclosporin oder Derivat davon oder eine Kombination daraus zur Verwendung vor einer Behandlung mit einem therapeutischen Wirkstoff, wobei der therapeutische Wirkstoff ein Chemotherapeutikum, ein antivirales Mittel oder ein Immunmodulator ist, bei einem Verfahren zur prophylaktischen Behandlung von Keratokonjunktivitis, die mit der Verwendung des therapeutischen Wirkstoffs assoziiert ist.

2. Cyclosporin zur Verwendung gemäß Anspruch 1, worin das Cyclosporin Cyclosporin A ist.

## Revendications

1. Cyclosporine ou dérivé de celle-ci, ou combinaison de celle-ci, pour une utilisation préalablement au traitement avec un agent actif sur le plan thérapeutique, ledit agent actif sur le plan thérapeutique étant un agent de chimiothérapie, un agent antiviral ou un immunomodulateur, dans un procédé de traitement prophylactique de la kératoconjonctivite associée à l'utilisation dudit agent actif sur le plan thérapeutique.

2. Cyclosporine pour une utilisation selon la revendication 1, dans laquelle ladite cyclosporine est la cyclosporine A.
